# EUROPEAN PATENT SPECIFICATION

(11) **EP 2 040 769 B1**
(45) Date of publication and mention of the grant of the patent: **26.02.2014**
(21) Application number: 07794783.6
(22) Date of filing: 11.05.2007
(51) Int. Cl.: A61L 27/34, A61L 31/10, A61L 27/54, A61L 31/16

(54) **CONTROL REALEASE DRUG COATING FOR MEDICAL DEVICES**
ARZNEIBESCHICHTUNG MIT KONTROLLIERTER FREISETZUNG FÜR MEDIZINPRODUKTE
REVÊTEMENT COMPRENANT UN MÉDICAMENT À LIBÉRATION CONTRÔLÉE POUR DES DISPOSITIFS MÉDICAUX

(30) Priority: 22.06.2006 US 472464
(43) Date of publication of application: 01.04.2009
(73) Proprietor: Boston Scientific Limited, Christ Church (BB)
(72) Inventor: KANGAS, Steve, Woodbury, MN 55125 (US)
(74) Representative: Lang, Johannes
(86) International application number: PCT/US2007/011410
(87) International publication number: WO 2007/149161

(56) References cited:
- EP-A- 1 588 725
- WO-A-02/26280
- US-A1- 2005 025 799
- US-A1- 2005 129 731
- US-B1- 6 306 419

## Description

### FIELD OF THE INVENTION

The present invention relates to drug-coated medical devices and methods of controlling the drug release profile from the same.

### BACKGROUND OF THE INVENTION

Medical devices may be coated with a polymer matrix into which a drug is dispersed. For example, many coronary stents are coated with a polymer containing a drug, such as paclitaxel. After the stent is placed inside the artery, the drug diffuses through the polymer matrix and is released into the surrounding tissue. The drug released from the stent can act to prevent re-occlusion of the artery after angioplasty and stent placement.

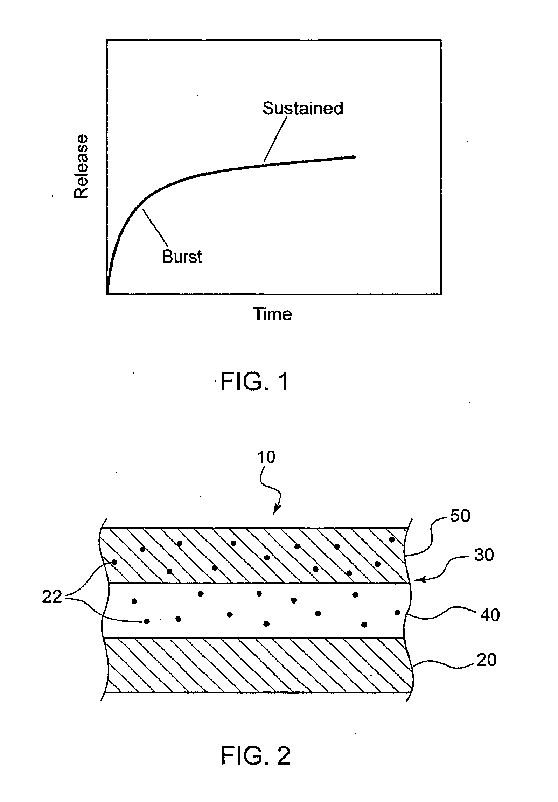

The rate at which the drug or therapeutic agent diffuses through the polymer matrix and is released into the surrounding fluid or tissue is characterized by its drug release profile. **FIG. 1** illustrates a representative drug release profile of a drug/polymer-coated stent where the drug is dispersed as a particulate in the polymer matrix. As indicated by **FIG. 1**, after implantation of the stent inside the target blood vessel, there is initially a rapid release of the drug residing at or near the surface of the polymer matrix (burst release) over a period of up to several days. This burst release is followed by a less rapid, sustained release phase over a period of many days, weeks, or months. This sustain release is due to the diffusion of drug from the interior of the polymer matrix.

It is desirable to control the drug release profile of a drug/polymer-coated stent, and this can be accomplished in various ways. Some approaches take advantage of the fact that drugs will diffuse through different polymers at different rates. For example, U.S. Patent No. 6,258,121 to Yang et al. describes forming a polymeric coating by blending together two different polymers. U.S. Patent No. 6,770,729 to Van Antwerp describes an approach in which multiple layers of very hydrophilic hydrogel polymers are used to control the drug release rate. Despite these and other approaches in the prior art, there continues to be a need for an approach to modulating drug release from a medical device in which the drug release profile can be better controlled.

US 2005/0025799 describes an implantable medical device having a multilayered coating, including a topcoat that can contain a drug.

US 2005/0129731 describes a dual layer stent coating having at least two polysulfone layers, which can be hydrophilic or hydrophobic to provide different elution kinetics.

EP 1588725 describes a stent having a coating of a heparin layer on top of a rapamycin layer.

WO 02/26280 describes biocompatible coatings and films for use on implantable medical devices, in which different polymer layers may be used to deliver different drugs or to control the release profile of a drug.

US 6,306,419 describes wound dressings and coated implantable medical devices that include a styrene sulfonate polymer.

### SUMMARY OF THE INVENTION

In one embodiment, the present invention provides a coated medical device comprising a medical device body and a coating disposed on the medical device body. The coating comprises a first layer and a second layer. The first layer comprises a first copolymer and a first therapeutic agent. The second layer comprises a second copolymer and a second therapeutic agent. The first and second copolymers are each selected from block copolymers, random copolymers, or alternating copolymers. The second layer is more hydrophilic than the first layer of the coating. The first layer is close to the medical body than the second layer. The first layer may comprise an unmodified copolymer, and the second layer may comprise a modified copolymer. Both the first and second layers may comprise both unmodified and modified copolymers with the ratios adjusted to achieve the desired release profile.

In another embodiment, the present invention provides a method of controlling the drug release profile from a coated medical device. The method comprises providing a coated medical device comprising a medical device body and a coating disposed on the medical device body. The coating comprises first and second layers, each of the first and second layers comprising block copolymers and a therapeutic agent, and wherein at least the second layer comprises a modified block copolymer. The first layer is closer to the medical device body than the second layer, and the second layer is more hydrophilic than the first layer.

Each layer may have both unmodified and modified copolymers. The ratio of the modified copolymers to the unmodified copolymers in each of the plurality of layers may be selected to control the release profile of the therapeutic agents from the coated medical device.

### BRIEF DESCRIPTION OF THE DRAWINGS

The present invention will become more fully understood from the detailed description given herein and the accompanying drawings which are given by way of illustration only, and thus do not limit the present invention, and wherein:
FIG. 1 illustrates the amount of drug released from a typical stent coating over time.
**FIG. 2** is a cross-sectional view of a fragmentary portion of a medical device according to an embodiment of the present invention showing a first and second layer of coating.
**FIG. 3** illustrates the cumulative percentage of paclitaxel released from a stent coated with maleic anhydride-grafted styrene-ethylene/butylene-styrene (SEBS) compared with a stent coated with unmodified styrene-isobutylene-styrene (SIBS).

### DETAILED DESCRIPTION OF THE INVENTION

Referring to **FIG. 2**, in one embodiment, the present invention provides a coated medical device **10** comprising a medical device body **20** and a coating **30** disposed on medical device body **20**. Coating **30**, in any of the embodiments of the present invention, can coat the entire surface of medical device body **20** or any portion less than the entire surface of medical device body **20**. As illustrated in **FIG. 2**, coating **30** comprises a first layer **40** and a second layer **50**. First and second layers **40 and 50** may comprise block copolymers, random copolymers, or alternating copolymers, and have a therapeutic agent or drug **22** dispersed therein (the terms "drug" and "therapeutic agent" are used interchangeably herein). According to the present invention, second layer **50** is more hydrophilic than first layer **40**. As used herein, the terms "hydrophilic" or "hydrophobic" are not intended to be restricted to absolute measures of water affinity. Rather, the terms are also used to describe relative water affinities. For example, although two different polymers may both be considered hydrophobic to one of ordinary skill in the art, one polymer may still be more hydrophilic than the other.

The release of drug from a polymer coating on a medical device is influenced by the hydrophilicity (or alternatively, hydrophobicity) of the polymer. Specifically, a relatively more hydrophilic polymer will allow a more rapid diffusion of fluid into the polymer matrix, causing a more rapid diffusion and release of drug than a less hydrophilic polymer. As used herein, the terms "slow," "fast," or "rapid" are not intended to be restricted to absolute measures of rate but rather are used to describe relative rates. Therefore, because second layer **50** is more hydrophilic than first layer **40**, according to this embodiment of the present invention, first and second layers **40** and **50** have different drug release profiles. Therapeutic agent **22** diffuses more rapidly from the copolymer of second layer **50** than the copolymer of first layer **40**, resulting in a relatively faster release of therapeutic agent **22** from second layer **50**. Similarly, because first layer **40** is less hydrophilic than second layer **50**, the therapeutic agent **22** diffuses more slowly through the copolymer of first layer **40** than through the copolymer of the second layer **50**, resulting in a relatively slower release of therapeutic agent from the first layer **40**. In the above-described embodiment, the relatively fast release of therapeutic agent **22** from second layer **50** represents the burst release of therapeutic agent **22** from medical device body **20**. Then, as therapeutic agent **22** from the second layer **50** becomes depleted, the drug release rate from the second layer **50** slows. However, the slower and continued release of therapeutic agent **22** from the first layer **40** provides for a sustained release of drug from medical device body **20**.

Of course, the above-described embodiment is only exemplary, and the drug release profile of medical device **10** can be controlled by altering the composition of coating **30**. For example, making second layer **50** more hydrophilic would increase the rate of drug diffusion from second layer **50** without substantially affecting drug diffusion from first layer **40**, thereby increasing the burst release without substantially affecting the sustained release. Alternatively, making the first layer **40** less hydrophilic would decrease the drug diffusion from first layer **40** without affecting drug diffusion from second layer **50**, thereby decreasing the sustained release without substantially affecting the burst release.

Although coating **30** is illustrated in **FIG. 1** as having only two layers, coating **30** may have any number of layers so long as one of the layers is more hydrophilic than another one of the layers and the layers are arranged in order of increasing hydrophilicity from the bottom to the top layer. In a preferred embodiment, coating **30** comprises a plurality of layers, with each layer having a different degree of hydrophilicity. For example, a medical device of the present invention may have three or more layers of coating with the top layer being relatively more hydrophilic, the middle layer being less hydrophilic, and the bottom layer being least hydrophilic.

The term "block copolymer" as used herein refers to a polymer having two or more different types of monomers joined together in the same polymer chain wherein blocks of monomers are grouped together. The term "block copolymer" encompasses both unmodified block copolymers and modified block copolymers. An "unmodified" block copolymer or "base" block copolymer used in the present invention is water insoluble and is generally considered to be hydrophobic with less than approximately 5% water pickup by weight. In one embodiment of the present invention, unmodified or base block copolymers can be those described in U.S. Patent No. 6,545,097 to Pinchuk et al.*,* Pinchuk describes block copolymers having the formula: (i) BAB or ABA (linear triblock); or (ii) B(AB)ₙ or A(BA)ₙ (linear alternating triblock); or (iii) X-(AB)ₙ or X-(BA)ₙ, where A is an elastomeric block such as a polyolefin, B is a thermoplastic block such as a vinyl aromatic or a methacrylate, X is a seed molecule, and n is a positive whole number. Examples of such block copolymers include styrene-isobutylene-styrene (SIBS) or styrene-ethylene/butylene-styrene (SEBS) which have a water pickup of approximately 1% by weight.

A "modified" copolymer used in the present invention is a more hydrophilic version of a base copolymer. A modified copolymer can be obtained through chemical addition of hydrophilic functional groups onto a base copolymer, or by synthesis using hydrophilic derivatives of the starting monomers of a base copolymer.. Examples of modified block copolymers obtained through chemical addition include sulfonated SIBS, maleic anhydride-grafted SIBS, sulfonated SEBS, and maleic anhydride-grafted SEBS. Examples of modified bock copolymers prepared using hydrophilic derivatives of the starting monomers of a base block copolymer include hydroxystyrene-isobutylene-hydroxystyrene and acetoxystyrene-isobutylene-acetoxystyrene.

The term "alternating copolymer" as used herein refers to a polymer having two or more different types of monomers joined together in the same polymer chain wherein the different monomers are arranged in an alternating order. The term "alternating copolymer" encompasses both unmodified alternating copolymers and modified alternating copolymers. An "unmodified" alternating copolymer or "base" alternating copolymer used in the present invention is water insoluble and is generally considered to be hydrophobic with less than approximately 5% water pickup by weight.

The term "random copolymer" as used herein refers to a polymer having two or more different types of monomers joined together in the same polymer chain wherein the different monomers may be arranged in any order. The term "random copolymer" encompasses both unmodified random copolymers and modified random copolymers. An "unmodified" random copolymer or "base" random copolymer used in the present invention is water insoluble and is generally considered to be hydrophobic with less than approximately 5% water pickup by weight. Examples of unmodified random copolymers include styrene-butadiene random copolymers and methylmethacrylate-butylacrylate random copolymers. Examples of modified random copolymers include hydroxystyrene-butadiene random copolymers and methylmethacrylate-butylacrylate-hydroxyethylmethacrylate random copolymers.

In another embodiment of the present invention, second layer **50** of coating **30** comprises a modified block copolymer and first layer **40** comprises an unmodified block copolymer. In such an embodiment, second layer **50** is more hydrophilic than first layer **40** and therefore has a faster drug release rate than first layer **40**. In still another embodiment of the present invention, first and second layers **40** and **50** can each comprise various blends of modified and unmodified block copolymers in various ratios. For example, the ratio of modified block copolymer to unmodified block copolymer in second layer **50** can be higher than the ratio of modified block copolymer to unmodified block copolymer in first layer **40** such that second layer **50** is more hydrophilic and thus, has a faster drug release profile than first layer **40**. As with the other embodiments of the present invention, coating **30** can include more than two layers with varying ratios of unmodified to modified block copolymers.

In another embodiment, the present invention provides a method of controlling the drug release profile from a coated medical device comprising the steps of providing a medical device, which comprises a medical device body, and providing a coating disposed on the medical device body. The coating comprises a plurality of layers, each of the plurality of layers comprising modified block copolymers, unmodified block copolymers, and therapeutic agents. According to this embodiment, the ratio of the modified block copolymers to unmodified block copolymers in each of the plurality of layers is varied to control the release profile of the therapeutic agents from the coated medical device. For example, the ratio can sequentially decrease from the topmost one of the plurality of layers to the bottommost one of the plurality of layers, resulting in sequentially slower drug release from the topmost layer to the bottommost layer.

One of skill in the art will appreciate that the present invention allows the release profile to be specifically tailored. For example, the burst release profile can be changed by altering the composition of one layer without substantially affecting the sustained release profile, and vice versa. In any of the embodiments of the present invention, other means can be employed to provide additional means to control the drug release profile of the medical device of the present invention. For example, because drug release from a polymer layer is influenced by the thickness of the layer and the drug:polymer ratio, the drug release profile of the medical device of the present invention can be controlled by varying the thickness or the drug:polymer ratio of any one or more of the layers of the coating.

In any of the embodiments of the present invention, non-limiting examples of block copolymers suitable for use include styrene-isobutylene-styrene (SIBS), styrene-ethylene/butylene-styrene (SEBS); hydroxysytrene-isobutylene-hydroxystyrene; a polyolefin elastomeric block; a thermoplastic block such as a vinyl aromatic block or a methacrylate block; and any combinations thereof. The block copolymers in any of the layers of a coating of a medical device of the present invention can be the same or different base block copolymers. Block copolymers can be modified using methods well known in the art such as grafting a maleic anhydride onto the block copolymer or sulfonating the block copolymer. For example, maleic anhydride can be grafted onto SEBS by mixing SEBS, maleic anhydride and an organic free radical initiator in an organic solvent, such as toluene or tetrahydrafuran. In another example, SIBS can be sulfonated by mixing SIBS, sulfuric acid and acetic anhydride in an organic solvent. The base block copolymers themselves can be made using methods well known in the art, such as the methods described in U.S. Patent No. 6,545,097 to Pinchuk et al..

In any of the embodiments of the present invention, the coating on the medical device can comprise additional layers positioned anywhere relative to the above-described layers of a coating. For example, a coating can further comprise a topmost biodegradable layer or a bottommost biostable layer. Further, any of the layers of a coating comprising a block copolymer can be blended with another non-biodegradable and/or biodegradable polymers. Non-limiting examples of suitable non-biodegradable polymers which could be incorporated into any of the plurality of layers include polystyrene; polystyrene-maleic anhydride; polyisobutylene copolymers; polyvinylpyrrolidone including cross-linked polyvinylpyrrolidone; polyvinyl alcohols, copolymers of vinyl monomers such as EVA; polyvinyl ethers; polyvinyl aromatics; polyethylene oxides; polyesters including polyethylene terephthalate; polyamides; polyacrylamides including poly(methylmethacrylate-butylacetate-methylmethacrylate) triblock copoylmers; polyethers including polyether sulfone; polyalkylenes including polypropylene, polyethylene and high molecular weight polyethylene; polyurethanes; polycarbonates, silicones; siloxane polymers; cellulosic polymers such as cellulose acetate; polymer dispersions such as polyurethane dispersions (BAYHDROL®); squalene emulsions; mixtures and copolymers of any of the foregoing; and hydrophilically modified polymers of any of the foregoing.

Non-limiting examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L,-lactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

One of skill in the art will appreciate that each layer of a coating of a medical device of the present invention may be applied by various means, such as spraying or dipping. Drying in between layers may or may not be necessary. One of skill in the art will also appreciate that there are various methods of preparing the coating mixture. For example, various solvents may be used (such as toluene or tetrahydrafuran), along with various therapeutic agents (such as paclitaxel) in various concentrations. Such coatings used with the present invention may be formed by any method known to one in the art. For example, an initial polymer/solvent mixture can be formed and then the therapeutic agent added to the polymer/solvent mixture. Alternatively, the polymer, solvent, and therapeutic agent can be added simultaneously to form the mixture. The polymer/solvent/therapeutic agent mixture may be a dispersion, suspension or a solution. The therapeutic agent may also be mixed with the polymer in the absence of a solvent. The therapeutic agent may be dissolved in the polymer/solvent mixture or in the polymer to be in a true solution with the mixture or polymer, dispersed into fine or micronized particles in the mixture or polymer, suspended in the mixture or polymer based on its solubility profile, or combined with micelle-forming compounds such as surfactants or adsorbed onto small carrier particles to create a suspension in the mixture or polymer. The coating may comprise multiple polymers and/or multiple therapeutic agents.

In other embodiments, different layers of a coating of a medical device of the present invention may have different drugs or therapeutic agents. It may be desirable to have different drugs released at different time periods. For example, second layer 50 may have one type of drug, for rapid release, that is beneficial early in the healing process after angioplasty, and first layer 40 may have another type of drug, for sustained or delayed release, that is beneficial later in the healing process. In other embodiments, different layers of a coating of a medical device of the present invention may have different concentrations of drug.

The therapeutic agent in a coating of a medical device of the present invention may be any pharmaceutically acceptable agent such as a non-genetic therapeutic agent, a biomolecule, a small molecule, or cells.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); anti-proliferative agents such as enoxaprin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl)ethyleneglycol-N,N,N',N'-tetraacetic acid and mixtures thereof; antibiotics such as gentamycin, rifampin, minocyclin, and ciprofolxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; bAR kinase (bARKct) inhibitors; phospholamban inhibitors; protein-bound particle drugs such as ABRAXANE™; and any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins ("MCP-1) and bone morphogenic proteins ("BMP's"), such as, for example, BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15. Preferred BMPS are any of BMP-2, BMP-3, BMP-4, BMP-5, BMP-6, and BMP-7. These BMPs can be provided as homdimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factor a and β, platelet-derived endothelial growth factor, platelet-derived growth factor, tumor necrosis factor α, hepatocyte growth factor, and insulin like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase ("TK") and combinations thereof and other agents useful for interfering with cell proliferation.

Exemplary small molecules include hormones, nucleotides, amino acids, sugars, and lipids and compounds have a molecular weight of less than 100kD.

Exemplary cells include progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin⁻) cells including Lin⁻ CD34⁻, Lin⁻CD34⁺, Lin⁻cKit⁺, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, immunologically masked cells, and teratoma derived cells.

Any of the therapeutic agents may be combined to the extent such combination is biologically compatible. A medical device of the present invention may also contain a radio-opacifying agent within its structure to facilitate viewing the medical device during insertion and at any point while the device is implanted. Non-limiting examples of radio-opacifying agents are bismuth subcarbonate, bismuth oxychloride, bismuth trioxide, barium sulfate, tungsten, and mixtures thereof.

### Examples

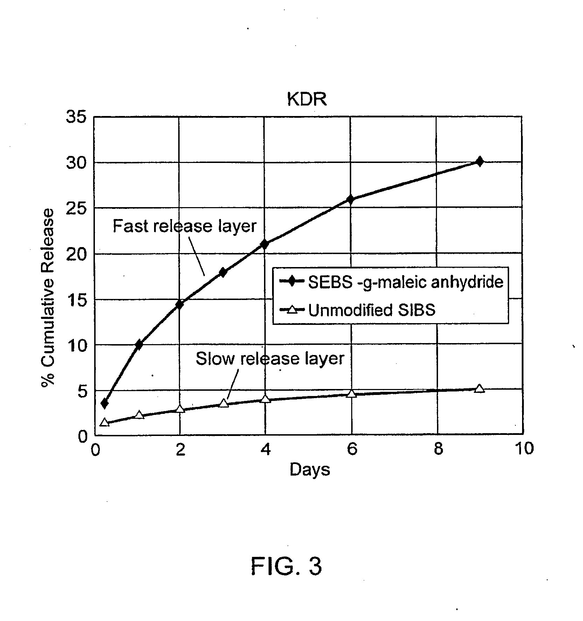

Unmodified SIBS and 8.8 wt % of paclitaxel were dissolved in an organic solvent to form a coating mixture. This coating mixture is sprayed onto a stent and allowed to dry. Subsequently, the amount of paclitaxel released from the stent is determined by alcohol and water extraction at 50°C, which is plotted on the line labeled "slow release layer" in **FIG. 3**. This release profile shows the cumulative amount of paclitaxel released from this stent coating over a period of time.

Maleic-anhydride SEBS and 8.8 wt % paclitaxel were dissolved in an organic solvent to form a coating mixture. This coating mixture was sprayed onto a stent and allowed to dry. Subsequently, the amount of paclitaxel released from the stent was determined by alcohol and water extraction at 50°C, which is plotted on the line labeled "fast release layer" in **FIG. 3**. Comparing these two drug release profiles demonstrates that there is a faster release of paclitaxel from the maleic anhydride-grafted SEBS polymer layer than from the unmodified SIBS polymer layer.

## Claims

1. A coated medical device comprising:
(a) a medical device body; and
(b) a coating disposed over the medical device body, the coating comprising:
(i) a first layer comprising a first copolymer having a first therapeutic agent therein; and
(ii) a second layer comprising a second copolymer having a second therapeutic agent therein, wherein the first layer is closer to the medical device body than the second layer, and wherein the second layer is more hydrophilic than the first layer;
wherein the first copolymer and second copolymer are each selected from the group consisting of block copolymers, random copolymers, or alternating copolymers.

2. The coated medical device of claim 1, wherein the coated medical device is a stent.

3. The coated medical device of claim 1, wherein the second copolymer is a modified block copolymer.

4. The coated medical device of claim 3, wherein the modified block copolymer is selected from the group consisting of sulfonated styrene-isobutylene-styrene, maleic anhydride-grafted styrene-isobutylene-styrene, sulfonated styrene-ethylene/butylene-styrene, and maleic anhydride-grafted styrene-ethylene/butylene-styrene, hydroxystyrene-isobutylene-hydroxystyrene, and acetoxystyrene-isobutylene-acetoxystyrene.

5. The coated medical device of claim 3, wherein the first copolymer is an unmodified block copolymer.

6. The coated medical device of claim 5, wherein the first layer further comprises a modified block copolymer.

7. The coated medical device of claim 3, wherein the second layer further comprises an unmodified block copolymer.

8. The coated medical device of claim 7, wherein the first copolymer is an unmodified block copolymer.

9. The coated medical device of claim 8, wherein the first layer further comprises a modified block copolymer.

10. The coated medical device of claim 9, wherein the ratio of the modified block copolymer to the unmodified block copolymer in the second layer is higher than the ratio of the modified block copolymer to the unmodified block copolymer in the first layer.

11. The coated medical device of claim 1, wherein the first copolymer is an unmodified block copolymer.

12. The coated medical device of claim 11, wherein the unmodified block copolymer is selected from the group consisting of styrene-isobutylene-styrene and styrene-ethylene/butylene-styrene.

13. The coated medical device of claim 1, wherein the coating further comprises an outermost layer comprising a biodegradable polymer.

14. The coated medical device of claim 1, wherein the first therapeutic agent and the second therapeutic agent are different.

15. The coated medical device of claim 1, wherein the first and second copolymers are block copolymers, and the first block copolymer, the second block copolymer, or both comprise an elastomeric block or a thermoplastic block.

16. A method of controlling the drug release profile from a coated medical device comprising:
(a) providing a medical device comprising a medical device body; and
(b) disposing a coating over the medical device body, the coating comprising first and second layers, each of the first and second layers comprising block copolymers and a therapeutic agent, wherein at least the second layer comprises a modified block copolymer, wherein the first layer is closer to the medical device body than the second layer, and wherein the second layer is more hydrophilic than the first layer.

17. The method of claim 16, wherein each of the first and second layers comprises a modified block copolymer and an unmodified block copolymer, and wherein the ratio of the modified block copolymer to the unmodified block copolymer in the second layer is greater than the ratio of the modified block copolymer to the unmodified block copolymer in the first layer.

18. The method of claim 16, wherein the therapeutic agent in the first layer is different from the therapeutic agent in the second layer.

19. The method of claim 16, wherein the block copolymer in at least one layer comprises an elastomeric block or a thermoplastic block.

## Patentansprüche

1. Eine beschichtete medizinische Vorrichtung umfassend:
(a) einen medizinischen Vorrichtungskörper; und
(b) eine Beschichtung, die über den medizinischen Vorrichtungskörper angeordnet ist, wobei die Beschichtung umfasst:
(i) eine erste Schicht, die ein erstes Copolymer mit einem ersten Therapeutikum darin umfasst; und
(ii) eine zweite Schicht, die ein zweites Copolymer mit einem zweiten Therapeutikum darin umfasst, wobei die erste Schicht näher an dem medizinischen Vorrichtungskörper ist als die zweite Schicht, und wobei die zweite Schicht hydrophiler ist als die erste Schicht;
wobei das erste Copolymer und das zweite Copolymer jeweils aus einer Gruppe ausgewählt werden, die aus Block-Colpolymeren, Random Copolymeren oder alternierenden Copolymeren besteht.

2. Beschichtete medizinische Vorrichtung nach Anspruch 1, wobei die beschichtete medizinische Vorrichtung ein Stent ist.

3. Beschichtete medizinische Vorrichtung nach Anspruch 1, wobei das zweite Copolymer ein modifiziertes Block-Copolymer ist.

4. Beschichtete medizinische Vorrichtung nach Anspruch 3, wobei das modifizierte Block-Copolymer aus der Gruppe ausgewählt wird, die aus sulfoniertem Styren-Isobutylen-Styren, Maleinsäureanhydrid gepfropftes Styren-Isobutylen-Styren, sulfonierte Styren-Ethylen/Butylen-Styren, und Maleinsäureanhydrid gepropftes Styren-Ethylen/Butylen-Styren, Hydroxystyren-Isobutylen-Hydroxystyren und Acetoxystyren-Isobutylen-Acetoxystyren, besteht.

5. Beschichtete medizinische Vorrichtung nach Anspruch 3, wobei das erste Copolymer ein unmodifiziertes Block-Copolymer ist.

6. Beschichtete medizinische Vorrichtung nach Anspruch 5, wobei die erste Schicht weiter ein modifiziertes Block-Copolymer umfasst.

7. Beschichtete medizinische Vorrichtung nach Anspruch 3, wobei die zweite Schicht weiter ein unmodifiziertes Block-Copolymer umfasst.

8. Beschichtete medizinische Vorrichtung nach Anspruch 7, wobei das erste Copolymer ein unmodifiziertes Block-Copolymer ist.

9. Beschichtete medizinische Vorrichtung nach Anspruch 8, wobei die erste Schicht weiter ein modifiziertes Block-Copolymer umfasst.

10. Beschichtete medizinische Vorrichtung nach Anspruch 9, wobei das Verhältnis des modifizierten Block-Copolymers zu dem unmodifizierten Block-Copolymer in der zweiten Schicht höher ist als das Verhältnis des modifizierten Block-Copolymers zum unmodifizierten Block-Copolymer in der ersten Schicht.

11. Beschichtete medizinische Vorrichtung nach Anspruch 1, wobei das erste Copolymer ein unmodifiziertes Block-Copolymer ist.

12. Beschichtete medizinische Vorrichtung nach Anspruch 11, wobei das unmodifizierte Block-Copolymer aus der Gruppe ausgewählt wird, die aus Styren-Isobutylen-Styren und Styren-Ethylen/Butylen-Styren besteht.

13. Beschichtete medizinische Vorrichtung nach Anspruch 1, wobei die Beschichtung weiter eine äußerste Schicht umfasst, die ein biologisch abbaubares Polymer umfasst.

14. Beschichtete medizinische Vorrichtung nach Anspruch 1, wobei das erste Therapeutikum und das zweite Therapeutikum unterschiedlich sind.

15. Beschichtete medizinische Vorrichtung nach Anspruch 1, wobei die ersten und zweiten Copolymere Block-Copolymere sind, und das erste Block-Copolymer, das zweite Block-Copolymer oder beide einen elastomeren Block oder einen thermoplastischen Block umfassen.

16. Ein Verfahren zum Kontrollieren des Arzneimittelfreisetzungsprofils von einer beschichteten medizinischen Vorrichtung umfassend:
(a) Bereitstellen einer medizinischen Vorrichtung, die einen medizinischen Vorrichtungskörper umfasst; und
(b) Anordnen einer Beschichtung über den medizinischen Vorrichtungskörper, wobei die Beschichtung erste und zweite Schichten umfasst, wobei jede der ersten und zweiten Schichten Block-Copolymere und ein Therapeutikum umfasst, wobei zumindest die zweite Schicht ein modifiziertes Block-Copolymer umfasst, wobei die erste Schicht näher an dem medizinischen Vorrichtungskörper ist als die zweite Schicht, und wobei die zweite Schicht hydrophiler ist als die erste Schicht.

17. Verfahren nach Anspruch 16, wobei jede der ersten und zweiten Schichten ein modifiziertes Block-Copolymer und ein unmodifiziertes Block-Copolymer umfasst, und wobei das Verhältnis von dem modifizierten Block-Copolymer zu dem unmodifizierten Block-Copolymer in der zweiten Schicht größer ist als das Verhältnis von dem modifizierten Block-Copolymer zu dem unmodifizierten Block-Copolymer in der ersten Schicht.

18. Verfahren nach Anspruch 16, wobei sich das Therapeutikum in der ersten Schicht von dem Therapeutikum in der zweiten Schicht unterscheidet.

19. Verfahren nach Anspruch 16, wobei das Block-Copolymer in zumindest einer Schicht einen elastomeren Block oder einen thermoplastischen Block umfasst.

## Revendications

1. Dispositif médical enrobé, comprenant :
(a) un corps de dispositif médical ; et
(b) un enrobage disposé sur le corps du dispositif médical, l'enrobage comprenant :
(i) une première couche comprenant un premier copolymère contenant un premier agent thérapeutique ; et
(ii) une deuxième couche comprenant un deuxième copolymère contenant un deuxième agent thérapeutique, la première couche étant plus proche du corps du dispositif médical que la deuxième couche, et la deuxième couche étant plus hydrophile que la première couche ;
le premier copolymère et le deuxième composition étant chacun choisi dans le groupe consistant en les copolymères à blocs, les copolymères aléatoires ou les compositions alternés.

2. Dispositif médical enrobé selon la revendication 1, le dispositif médical enrobé étant un stent.

3. Dispositif médical enrobé selon la revendication 1, dans lequel le deuxième copolymère est un copolymère à blocs modifié.

4. Dispositif médical enrobé selon la revendication 3, dans lequel le copolymère à blocs modifié est choisi dans le groupe consistant en le styrène sulfoné-isobutylène-styrène, l'anhydride maléique-styrène greffé-isobutylène-styrène, le styrène sulfoné-éthylène/butylène-styrène, et l'anhydride maléique-styrène greffé-éthylène-butylène-styrène, l'hydroxystyrène-isobutylène-hydroxystyrène et l'acétoxystyrène-isobutylène-acétoxystyrène.

5. Dispositif médical enrobé selon la revendication 3, dans lequel le premier copolymère est un copolymère à blocs non modifié.

6. Dispositif médical enrobé selon la revendication 5, dans lequel la première couche comprend en outre un copolymère à blocs modifié.

7. Dispositif médical enrobé selon la revendication 3, dans lequel la deuxième couche comprend en outre un copolymère à blocs non modifié.

8. Dispositif médical enrobé selon la revendication 7, dans lequel le premier copolymère est un copolymère à blocs non modifié.

9. Dispositif médical enrobé selon la revendication 8, dans lequel la première couche comprend en outre un copolymère à blocs modifié.

10. Dispositif médical enrobé selon la revendication 9, dans lequel le rapport entre le copolymère à blocs modifié et le copolymère à blocs non modifié dans la deuxième couche est supérieur au rapport entre le copolymère à blocs modifié et le copolymère à blocs non modifié dans la première couche.

11. Dispositif médical enrobé selon la revendication 1, dans lequel le premier copolymère est un copolymère à blocs non modifié.

12. Dispositif médical enrobé selon la revendication 11, dans lequel le copolymère à blocs non modifié est choisi dans le groupe consistant en le styrène-isobutylène-styrène et le styrèneéthylène/butylène-styrène.

13. Dispositif médical enrobé selon la revendication 1, dans lequel l'enrobage comprend en outre une couche la plus extérieure, comprenant un polymère biodégradable.

14. Dispositif médical enrobé selon la revendication 1, dans lequel le premier agent thérapeutique et le deuxième agent thérapeutique sont différents.

15. Dispositif médical enrobé selon la revendication 1, dans lequel le premier et le deuxième copolymères sont des copolymères à blocs, et le premier copolymère à blocs, le deuxième copolymère à blocs, ou les deux, comprennent un bloc élastomère ou un bloc thermoplastique.

16. Procédé de régulation du profil de libération d'un médicament à partir d'un dispositif médical enrobé, comprenant :
(a) la mise à disposition d'un dispositif médical comprenant un corps de dispositif médical ; et
(b) la mise en place d'un enrobage sur le corps du dispositif médical, l'enrobage comprenant une première et une deuxième couches, chacune de la première et de la deuxième couches comprenant des copolymères à blocs et un agent thérapeutique, au moins la deuxième couche comprenant un copolymère à blocs modifié, la première couche étant plus proche du corps du dispositif médical que la deuxième couche, et la deuxième couche étant plus hydrophile que la première couche.

17. Procédé selon la revendication 16, dans lequel chacune de la première et de la deuxième couches comprend un copolymère à blocs modifié et un copolymère à blocs non modifié, et dans lequel le rapport entre le copolymère à blocs modifié et le copolymère à blocs non modifié dans la deuxième couche est supérieur au rapport entre le copolymère à blocs modifié et le copolymère à blocs non modifié dans la première couche.

18. Procédé selon la revendication 16, dans lequel l'agent thérapeutique dans la première couche est différent de l'agent thérapeutique dans la deuxième couche.

19. Procédé selon la revendication 16, dans lequel le copolymère à blocs dans au moins une couche comprend un bloc élastomère ou un bloc thermoplastique.
